# EUROPEAN PATENT SPECIFICATION

(11) **EP 2 667 922 B1**
(45) Date of publication and mention of the grant of the patent: **15.04.2020**
(21) Application number: 12706205.7
(22) Date of filing: 27.01.2012
(51) Int. Cl.: A61M 25/02, A61F 13/15

(54) **SHIELD APPARATUS**
ABSCHIRMUNGSVORRICHTUNG
APPAREIL DE PROTECTION

(30) Priority: 27.01.2011 GB 201101402; 16.02.2011 GB 201102690
(43) Date of publication of application: 04.12.2013
(73) Proprietor: Prosys International Ltd, London SW20 0TB (GB)
(72) Inventor: STEER, Graham, London SW20 0TB (GB)
(74) Representative: Milhench, Mark Lorne
(86) International application number: PCT/EP2012/051365
(87) International publication number: WO 2012/101265

(56) References cited:
- EP-A1- 0 807 449
- WO-A2-2007/011596
- US-A- 5 605 546
- US-A1- 2008 077 064
- US-A1- 2010 294 286

## Description

### Field

The present invention relates to apparatus that is configured to be worn by a person to whom a catheter (such as a central venous catheter) has been fitted. In one illustrative application, use of such an apparatus reduces the likelihood of water coming into contact with a person's catheter while they shower.

### Background

Central venous catheters (CVCs) are configured to be in direct communication with the bloodstream of a patient. As such, the risk of infection and blood poisoning to a person wearing a CVC is significantly increased. Alarmingly, the article "Keeping Central Line Infections at Bay" Nursing 36(4):58-64, April 2006 by L. Hadayaw reports that the mortality rate for CVC related bloodstream infections is estimated to be between 4% and 20%. Blood poisoning is typically caused by substances and foreign bodies coming into contact with the site in a person's body through which a CVC protrudes (typically referred to as a catheter exit site). One such substance that could carry pathogens and/or bacteria into a CVC exit site is water, and because of this it is typically the case that patients to whom a CVC has been fitted are usually instructed not to take a shower or a bath.

Clearly the inability for a patient to take a shower or a bath causes disruption to their lifestyle as they are restricted to using less efficient methods of cleaning themselves. This problem is particularly acute for those patients, such as kidney dialysis patients, who are often fitted with a catheter for an extended period of time.

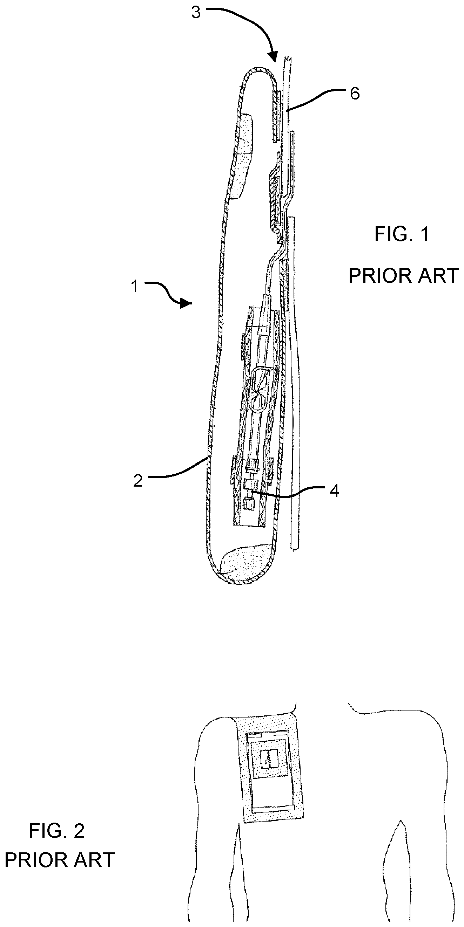

One previously proposed device that seeks to address this problem is disclosed in US Patent Application No. US2008/0208145. This document describes a disposable guard that a patient with a CVC wears around their catheter to enable them to take a shower. With reference to Fig. 1, such a guard 1 comprises a bag 2 into which the distal end of a catheter 4 is inserted. The bag 2 is then adhered to a person's body 6 around the exit site through which the catheter protrudes, thereby sealing the catheter 4 within the bag 2.

Whilst this arrangement does provide some measure of protection, problems may occur if the bag has not been properly sealed to the skin by the patient. For example, if there are hairs trapped between the bag 2 and the patient's skin 6 then there is the possibility that water may be able to seep through the seal and into the bag 2. Unfortunately, this problem is exacerbated by the fact that the bag design tends to cause water to pool between the top of the bag and the skin (i.e. in the region indicated by reference numeral 3) when a patient is standing in a shower.

Another device that seeks to address similar problems is disclosed in European Patent No. EP1420848. The device disclosed in this document also includes a bag into which the distal end of a catheter is inserted. The bag is then adhered to a patient's body around the entrance site of their catheter thus forming a first barrier for restricting water ingress into the bag. A flexible shield is then adhered to the patient's body around the aforementioned bag so that it completely encloses the bag and thereby forms a second barrier to water ingress.

Whilst this arrangement further reduces the chance of water entering the bag through the seal, the shield is necessarily quite large and as a consequence it can be difficult for a patient to manipulate the shield into position without assistance. This problem is exacerbated in cases where the CVC in their neck or upper chest, as part of the shield will extend over the shoulder (as shown in Fig. 2) or behind the head. A further problem associated with this arrangement is that as the shield is quite large (in any event larger than the bag into which the distal end of the catheter extends), it prevents the patient from washing a significant amount of their skin.

Aspects of the present invention have been devised with the foregoing problems in mind.

Other previously proposed devices are disclosed in WO2007/011596, US2008/077064, US5605546, EP0807449 and US2010/294286.

WO2007/011596 discloses a protective device having a substantially rigid casing for placement on a patient's skin, wherein the casing has a hollow interior facing the patient's skin and a lip. The casing can be spanned across its hollow interior by a membrane. The casing can have a first adhesive seal attached to a bottom surface of the lip of the casing, and a second adhesive seal attached to the top surface of the lip of the casing.

US2008/077064 discloses a bandage bag that comprises a bag and two adhesive layers. The bag opens along a first edge. One adhesive layer is placed along the other three edges on the anterior side of the bag. The second adhesive layer is placed along the first edge on the posterior side of the bag. The bag is attached to a patient's arm near the insertion site using the second adhesive layer. The catheter tubing and port is placed in the bag. The bag is then folded over so the first adhesive layer on the anterior side faces the arm and covers the insertion site, forming a waterproof bandage for the insertion site and the medical apparatus.

US5605546 discloses apparatus for protecting the outwardly extending portion of an indwelling medical treatment device, for example, a catheter. Such apparatus include an at least partially transparent receptacle defining a chamber sized and adapted to receive an outwardly extending portion of an indwelling medical treatment device, an inlet in the receptacle through which the outwardly extending portion is passed to be received by the chamber, and a securement member on the receptacle adapted to be secured to the human or animal patient so as to substantially prevent liquid contamination of the outwardly extending portion received in the chamber.

EP0807449 discloses a deep catheter dressing that consists of a pouch with an aperture to receive the end of at least one catheter. An adhesive mechanism attaches the pouch to the patient's skin. The adhesive mechanism surrounds the pouch and ensures that it is sealed.

US2010/294286 discloses a leak-resistant medical barrier that comprises a sheet with a first side and a second side. The first side has an adhesive portion adapted to be disposed on a patient's skin and a release liner disposed on the adhesive portion. The second side also has an adhesive portion adapted to be disposed either on a patient's skin, on itself, or on a patient's skin and on itself and a release liner disposed on its adhesive portion.

### Summary

In accordance with a presently preferred embodiment of the present invention, there is provided apparatus for shielding a catheter from contact with water falling from a shower head of a shower, the apparatus comprising: a first shield component having a first part that is configured to form a first seal around an exit aperture from which a catheter exits the patient's body, said first part defining an internal volume that opens to an internal volume of a bag-like second part that is configured to receive a distal part of said catheter; and a second shield component for adhering to the patient's body around at least part of the periphery of the first part of said first shield component so as to provide a second seal against water contact with said exit aperture; wherein said first and second shield components cooperate, when the apparatus is worn by a patient, to provide two seals against contact between water falling from said shower head and said exit aperture without obstructing access to an area of the patient's skin beneath said second part of said first shield component.

An advantage of this arrangement is that the apparatus covers a much smaller proportion of the patient's skin than the apparatus depicted in Fig. 2, and hence the patient is more easily able to affix the apparatus to their body without assistance. A further advantage of this arrangement is that as the catheter tails are wholly enclosed in the second portion of the first component and the second component does not overly that second portion, so a user wearing the apparatus whilst showering can lift the second portion and wash the skin that lies beneath that part first component. Hence a user is prevented from washing a far smaller area of their skin than they are with the device depicted in Fig. 2, for example. A further advantage of this arrangement is that water cannot pool between the first component and the patient's skin.

In a preferred arrangement the second shield component is configured to at least partially cover the first part of said first shield component.

The second shield component may be provided with adhesive for forming said second seal. In one envisaged arrangement, the adhesive provided on said second shield component is generally C-shaped.

In a preferred arrangement the first seal is located wholly within the C-shaped adhesive on said second shield component when the second component is fitted over the first shield component.

The first part of said first shield component preferably comprises an entry aperture for receiving said catheter.

The first shield component may comprise a liner accommodated within said aperture.

In one arrangement, substantially all of one face of said second shield component may be covered with adhesive.

The second part of said first shield component may be narrower than said first part of said first shield component.

In one particular arrangement the second shield component comprises first, second and third discrete cover components.

Such first, second and third cover components may each be configured to be coupled to the body of a patient and to the first part of the first shield component.

The first, second and third shield components may be configured to extend along respective edges of the first part of the first shield component when coupled thereto.

The third cover component may be configured to partially cover the first cover component and the second cover component when the first, second and third cover components are coupled to the first part of the first shield component.

Other features, aspects and advantages of the invention will be apparent from the following detailed description. References to embodiments throughout the description which are not under the scope of the appended claims merely represent possible exemplary executions and are therefore not part of the present invention.

### Brief Description of the Drawings

Various aspects of the teachings of the present invention, and arrangements embodying those teachings, will hereafter be described by way of illustrative example with reference to the accompanying drawings, in which:
Figs. 1 and 2 each show a prior art CVC shield device;
Fig. 3 is a schematic plan view of apparatus according to a first embodiment of the invention;
Fig. 4 is a schematic plan view of the apparatus shown in Fig 3 assembled in use;
Fig. 5 is a schematic plan view of apparatus according to a second embodiment of the invention;
Fig. 6 is a schematic plan view of apparatus according to a third embodiment of the invention;
Figs. 7 and 8 are schematic plan and sectional views, respectively, of a shield for use with any of the aforementioned embodiments; and
Figs. 9 to 11 are schematic plan views of apparatus according to a fourth embodiment of the invention.

### Detailed Description

Illustrative implementations of the teachings of the present invention will now be described with particular reference to shield apparatus for a central venous catheter (CVC) that typically has two or more lumens. However, it should be remembered that this particular implementation is merely illustrative, and that the teachings of the present invention may equally be applied to a shield apparatus for use with any other type of catheter.

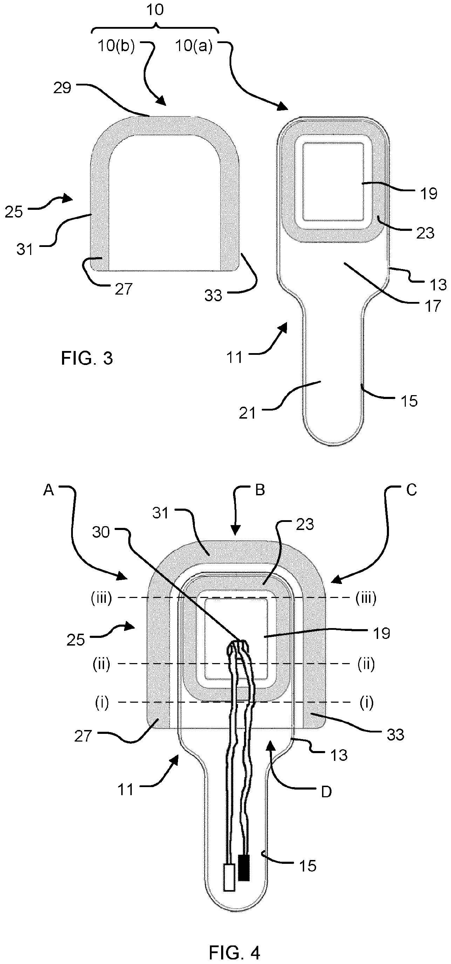

With the above proviso in mind, reference will now be made to Figs. 3 to 5 of the accompanying drawings in which there is depicted a shield apparatus 10 that embodies the teachings of the invention. The shield apparatus 10 is configured to be worn by a patient to whom a CVC catheter has been fitted, and functions to reduce the likelihood of shower water entering the body via the catheter exit site.

With particular reference to Fig. 3 the shield apparatus 10 comprises first 10(a) and second 10(b) shield components that cooperate, in a manner that is later described in detail, to reduce the likelihood of water from a shower coming into contact with the exit site of a catheter.

The first shield component 10(a) is generally in the form of a bag 11 that functions to receive, envelop and thereby protect a distal portion of a catheter (not shown) that protrudes from a user. In an envisaged arrangement the bag 11 comprises a first portion 13 and a second portion 15.

The first portion 13 defines an internal volume 17 that can be accessed via an entry aperture 19, and which opens to an internal volume 21 of the second portion 15. The internal volume 21 of the second portion 15 receives the distal end(s) of one or more lumens of a catheter when the apparatus is worn, and in a preferred implementation (shown in Fig. 3) the first portion 13 may be wider than the second portion 15 to facilitate insertion of the catheter distal ends into the internal volume 21 of the second portion 15. As will be appreciated by persons skilled in the art, when the distal ends are located in the internal volume of the second portion, they are protected by the second portion from contact with water.

The bag 11 is provided with an adhesive strip 23 that preferably extends substantially all of the way round the periphery of the entry aperture 19 so that a user can couple the bag 11 to their body in such a way that the first portion 13 overlies the catheter exit site, and the adhesive strip 23 forms a seal around the exit site. The adhesive strip is preferably provided with a cover (for example of waxed paper) that a user removes to expose the adhesive.

The second shield component comprises a guard 25 that includes an adhesive strip 27 which extends at least partway around the periphery of the guard. In a particularly preferred arrangement, the adhesive strip extends along a first side 29 of the guard, which first side 29 is uppermost when the apparatus is worn by a user. The adhesive strip may also extend from the first side at least partway down second and third parallel sides 31, 33 (which sides will be generally vertical when the guard is adhered to a patient with the first edge uppermost).

The guard 25 is shaped and configured to fit over at least part of the first portion 13 of the bag 11, and when so arranged the adhesive strip 27 of the guard 25 provides a second seal against water ingress from those directions (namely sideways and downwards) in which water is most likely to be to be flowing whilst a patient takes a shower).

Fig. 4 is a schematic plan view of the two components of the shield apparatus assembled in use with the guard 25 overlying part of the first portion 13 of the bag 11. As will be appreciated from Fig. 4, the apparatus provides two seals against sideways water ingress (i.e in directions A & C), and downwards water ingress (i.e. in direction B). The apparatus only provides a single seal against water ingress in an upwards direction (i.e. in direction D), but as water flows downwards from a shower head, it is anticipated that a single seal will be more than adequate to deal with any water that might inadvertently splash upwards whilst the person fitted with the catheter is showering.

In a particularly preferred arrangement, as shown in Fig. 4, the adhesive strip 27 of the guard 25 is generally U-shaped and extends down the second and third guard sides 31, 33 to a point below the seal formed by the adhesive strip 23 on the first component. In this configuration the adhesive strip 23 on the first component is wholly within the U-shaped adhesive strip 27 on the guard 25.

Whilst this arrangement is preferred, it will be appreciated that the adhesive strip 27 on the guard sides 31, 33 need not extend downwardly beyond the adhesive strip 23 on the first component, and could instead terminate at a higher point (i) in the vicinity of the adhesive strip 23 on the first component. Whilst it is particularly preferred for the adhesive strip 27 to at least extend to a point (ii) below the exit site 30, it will be appreciated by persons skilled in the art that an adhesive strip which terminated at or around point (iii) would still provide an arrangement that avoided many of the problems (in particular the aforementioned pooling problem) associated with the devices depicted in Figs. 1 and 2 of the drawings.

As a further means of avoiding problems associated with pooling of water it is particularly preferred for the adhesive strip 27 of at least the guard to extend inwardly from the peripheral edge(s) of the guard. The adhesive strip 23 of the bag 11 may likewise extend inwardly from the peripheral edges of the bag 11 towards the aperture 19, or may instead be spaced therefrom.

As can easily be appreciated by comparing Figs. 2 and 4, the apparatus depicted in Fig. 4 covers a much smaller proportion of the patient's skin than the apparatus depicted in Fig. 2, and hence the patient is more easily able to affix the apparatus to their body without assistance. A further advantage of the apparatus depicted in Fig. 4 is that as the catheter tails are wholly enclosed in the second portion of the bag and the guard 25 does not overly that second portion, so a user wearing the apparatus whilst showering can lift the second portion and wash the skin that lies beneath that part of the bag 11. Hence a user is prevented from washing a far smaller area of their skin than they are with the device depicted in Fig. 2, for example. A further advantage of the arrangement shown in Fig. 4 is that water cannot pool between the bag 11 and the adhesive strip 23 on the bag 11.

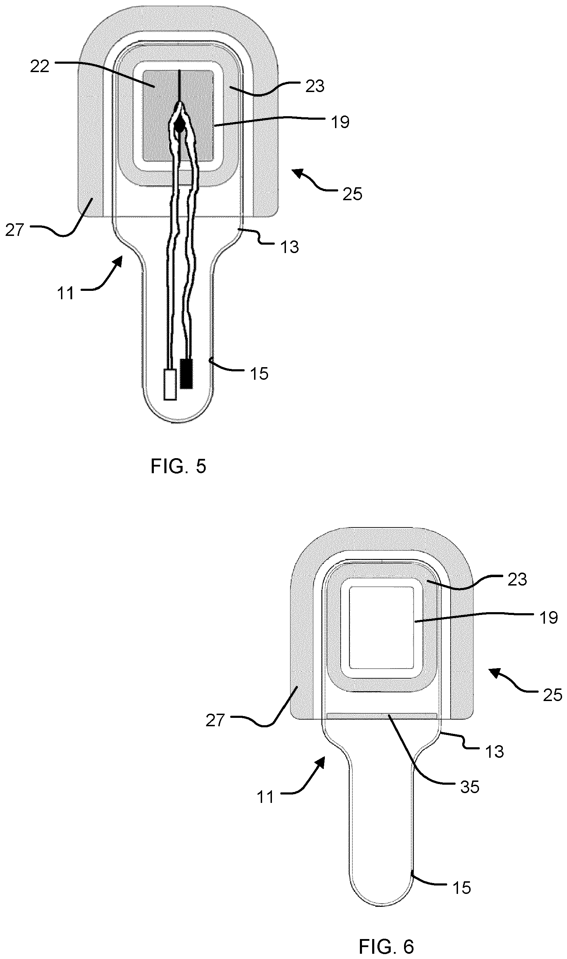

In the arrangement depicted in Fig. 4, the entry aperture 19 simply comprises a hole in the bag 11, but in a modification of this arrangement (shown in Fig. 5) the hole 19 in the bag 11 may be filled with an absorbent liner 22. Such a liner 22 may comprise a body of absorbent material with an opening (such as a slit) in the liner through which a user's catheter line extends when the catheter is inserted into the bag 11. As will be appreciated, the liner 22 provides a final line of protection to a user's catheter exit site by absorbing water that breaches the adhesive seal 23 provided on the bag 11. In another envisaged arrangement, the absorbent liner 22 may be coated and/or impregnated with steriliser for sterilising a user's catheter entrance site (such as an appropriate antiseptic or antibacterial substance for example).

In a further modification, depicted schematically in Fig. 6, the guard 25 may comprise a further strip of adhesive 35 that couples the guard 25 to the bag 11 when the guard 25 is positioned over the first part of the bag. In one envisaged arrangement the additional strip of adhesive may be located, as shown in Fig. 6, proximate a fourth edge of the guard 25 that is generally parallel to the first edge 29. As will be appreciated, the guard shown in Fig. 6 could be used with a bag of the type depicted in Fig. 5, and in a further modification the additional strip of adhesive could be provided on the bag 11.

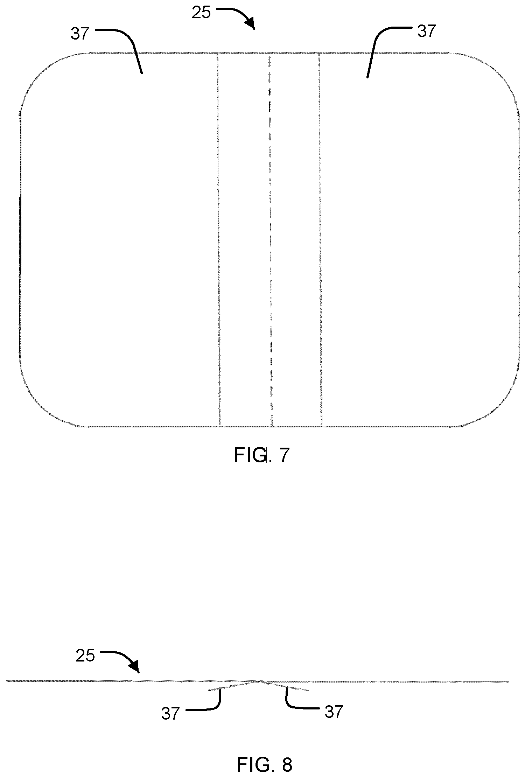

In yet another envisaged arrangement, substantially the entirety of the underside of the guard 25 may be provided with an adhesive so that the guard adheres to the skin and to the bag 11. In this arrangement the adhesive underside of the guard 25 may be provided, as shown in Figs. 7 and 8, with two waxed adhesive covers 37 that can be removed by a user to expose the adhesive.

In a preferred implementation the bag 11 and guard 25 are formed of flexible, water impervious material. For example the bag 11 and guard 25 may be formed of polyethylene.

It will be appreciated that whilst various aspects and embodiments of the present invention have heretofore been described, the scope of the present invention is not limited to the particular arrangements set out herein and instead extends to encompass all arrangements, and modifications and alterations thereto, which fall within the scope of the appended claims.

For example, in an envisaged arrangement the apparatus may be supplied to a patient with the guard and bag pre-coupled to one another, for example by welding or adhering the guard to the bag. In another envisaged arrangement, the guard and bag could be integrated into a single component, with the guard comprising a flap that extends beyond the periphery of the bag - the flap being provided with an adhesive strip for forming a seal against the patient's skin.

In yet another envisaged arrangement, the cover may comprise three discrete components, one for adhering to the patient so that it covers (and adheres to) an upper (typically generally horizontal) edge of the bag, and the other two components for adhering to the patient so that they cover (and adhere to) each lateral (typically generally vertical) side of the bag.

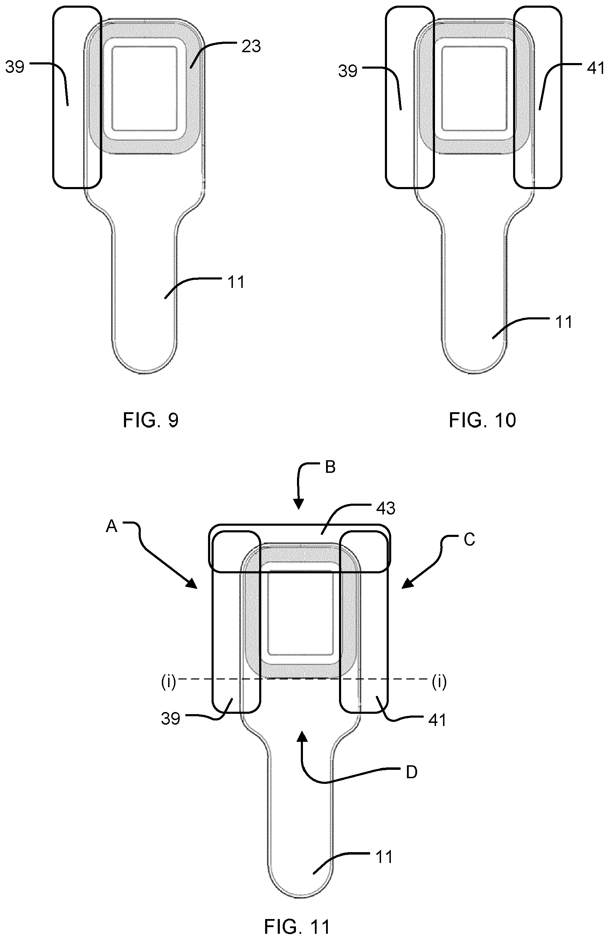

Fig. 9 illustrates a bag 11, in use, to which a first cover component 39 has been adhered (the or each catheter lumen being omitted from the drawing for clarity). The first cover component 39 is adhered to a patient's body and to the left-side generally vertical edge of the bag 11. In the arrangement shown in Fig. 9 the first cover 39 extends along the entirety of, and thereby covers, the left-side vertical portion of the adhesive 23.

Fig. 10 illustrates the bag 11 in Fig. 9 after a second cover component 41 has been adhered to the bag 11. The second cover component 41 is adhered to the patient's body and to the right-side generally vertical edge of the bag. In the arrangement shown in Fig. 10 the second cover component 41 extends along the entirety of, and thereby covers, the right-side vertical portion of the adhesive 23.

Fig. 11 illustrates the bag 11 in Fig. 10 after a third cover component 43 has been adhered to the bag 11. The third cover component 43 is adhered partially to the patient's body, partially to the upper generally horizontal edge of the bag 11, and partially to the first and second cover components 39, 41. In the arrangement shown in Fig. 11 the third cover component 43 extends along the entirety of, and thereby covers, the upper horizontal portion of the adhesive 23. The third cover component 43 also partially covers (at least the uppermost edges) of the first and second cover components 39, 41.

As with the arrangement illustrated in Fig. 4 it will be appreciated that the apparatus illustrated in Fig. 11 provides two seals against sideways water ingress (i.e in directions A & C), and downwards water ingress (i.e. in direction B). The apparatus only provides a single seal against water ingress in an upwards direction (i.e. in direction D), but as water flows downwards from a shower head, it is anticipated that a single seal will be more than adequate to deal with any water that might inadvertently splash upwards whilst the patient is showering.

Furthermore, as with the arrangement shown in Fig. 4 it will be appreciated that the first and second cover components 39, 41 need not extend downwardly beyond the adhesive strip 23 on the bag 11 (as shown in Fig. 11). The first and second cover components 39, 41 could instead terminate at a higher point (i) in the vicinity of the adhesive strip 23 on the bag. Whilst it is particularly preferred for the first and second cover components 39, 41 to at least extend to a point below a catheter exit site (not shown in Fig. 11 to enhance clarity of the drawing), it will be appreciated by persons skilled in the art that the first and second cover components 39, 41 may extend any length along the respective side edges of the bag 11 because this would still provide an arrangement that avoided many of the problems (in particular the aforementioned pooling problem) associated with the devices depicted in Figs. 1 and 2 of the drawings.

## Claims

1. Apparatus (10) for shielding a catheter from contact with water falling from a shower head of a shower, the apparatus comprising:
a first shield component (10a) having a first part (13) that is configured to form a first seal around an exit aperture (30) from which a catheter exits the patient's body, said first part (13) defining an internal volume (17) that opens to an internal volume (21) of a bag-like second part (15) that is configured to receive a distal part of said catheter; and
a second shield component (10b; 39, 41, 43) for adhering to the patient's body around at least part of the periphery of the first part (13) of said first shield component (10a) so as to provide a second seal against water contact with said exit aperture (30);
wherein said first (10a) and second (10b; 39, 41, 43) shield components cooperate, when the apparatus (10) is worn by a patient, to provide two seals against contact between water falling from said shower head and said exit aperture (30) without obstructing access to an area of the patient's skin beneath said second part (15) of said first shield component (10a).

2. The apparatus of Claim 1, wherein the second shield component (10b; 39, 41, 43) is configured to at least partially cover the first part (13) of said first shield component (10a).

3. The apparatus of Claim 1 or 2, wherein the second shield component (10b; 39, 41, 43) is provided with adhesive for forming said second seal.

4. The apparatus of Claim 3, wherein the adhesive (27) provided on said second shield component (10b) is generally C-shaped.

5. The apparatus of Claim 4, wherein the first seal is located wholly within the C-shaped adhesive (27) on said second shield component (10b) when the second component (10b) is fitted over the first shield component (10a).

6. The apparatus of any preceding claim, wherein the first part (13) of said first shield component (10a) comprises an entry aperture (19) for receiving said catheter.

7. The apparatus of Claim 6, wherein said first shield component (10a) comprises a liner (22) accommodated within said aperture.

8. The apparatus of any of any preceding claim, wherein substantially all of one face of said second shield component (10b) is covered with adhesive.

9. The apparatus of any preceding claim, wherein said second part (15) of said first shield component (10a) is narrower than said first part (13) of said first shield component (10a).

10. The apparatus of any of Claims 1 to 3, wherein the second shield component comprises a plurality of discrete cover components (39, 41, 43).

11. The apparatus of Claim 10, wherein the second shield component comprises first (39), second (41) and third (43) discrete cover components.

12. The apparatus of Claim 11, wherein the first, second and third cover components (39, 41, 43) are each configured to be coupled to the body of a patient and to the first part (13) of said first shield component (10a).

13. The apparatus of Claim 12, wherein the first, second and third shield components (39, 41, 43) are configured to extend along respective edges of the first part (13) of the first shield component (10a) when coupled thereto.

14. The apparatus of Claim 13, wherein the third cover component (43) is configured to partially cover the first cover component (39) and the second cover component (41) when the first, second and third cover components are coupled to the first part (13) of the first shield component (10a).

## Patentansprüche

1. Vorrichtung (10) zum Abschirmen eines Katheters vor einem Kontakt mit Wasser, das von einem Duschkopf einer Dusche fällt, wobei die Vorrichtung Folgendes umfasst:
eine erste Abschirmkomponente (10a), die einen ersten Teil (13) aufweist, der konfiguriert ist, um eine erste Abdichtung um eine Austrittsöffnung (30) zu bilden, von der ein Katheter den Körper des Patienten verlässt, wobei der erste Teil (13) ein Innenvolumen (17) definiert, das sich zu einem Innenvolumen (21) eines beutelartigen zweiten Teils (15) öffnet, der konfiguriert ist, um einen distalen Teil des Katheters aufzunehmen; und
eine zweite Abschirmkomponente (10b, 39, 41, 43) zum Haften an dem Körper des Patienten zumindest um einen Teil des Umfangs des ersten Teils (13) der ersten Abschirmkomponente (10a), um eine zweite Abdichtung gegenüber Wasserkontakt mit der Austrittsöffnung (30) bereitzustellen;
wobei die erste (10a) und zweite (10b; 39, 41, 43) Abschirmkomponente zusammenwirken, wenn die Vorrichtung (10) von einem Patienten getragen wird, um zwei Abdichtungen gegenüber einem Kontakt zwischen Wasser, das von dem Duschkopf fällt und der Austrittsöffnung (30) bereitzustellen, ohne den Zugriff auf einen Bereich der Haut des Patienten unter dem zweiten Teil (15) der ersten Abschirmkomponente (10a) zu behindern.

2. Vorrichtung nach Anspruch 1, wobei die zweite Abschirmkomponente (10b; 39, 41, 43) konfiguriert ist, um zumindest teilweise den ersten Teil (13) der ersten Abschirmkomponente (10a) zu bedecken.

3. Vorrichtung nach Anspruch 1 oder 2, wobei die zweite Abschirmkomponente (10b; 39, 41, 43) mit einem Klebstoff versehen ist, um die zweite Abdichtung zu bilden.

4. Vorrichtung nach Anspruch 3, wobei der an der zweiten Abschirmkomponente (10b) bereitgestellte Klebstoff (27) im Allgemeinen C-förmig ist.

5. Vorrichtung nach Anspruch 4, wobei sich die erste Abdichtung vollständig in dem C-förmigen Klebstoff (27) an der zweiten Abschirmkomponente (10b) befindet, wenn die zweite Komponente (10b) über die erste Abschirmkomponente (10a) gezogen wird.

6. Vorrichtung nach einem der vorhergehenden Ansprüche, wobei der erste Teil (13) der ersten Abschirmkomponente (10a) eine Eintrittsöffnung (19) zum Aufnehmen des Katheters umfasst.

7. Vorrichtung nach Anspruch 6, wobei die erste Abschirmkomponente (10a) eine Auskleidung (22) umfasst, die in der Öffnung aufgenommen wird.

8. Vorrichtung nach einem der vorhergehenden Ansprüche, wobei im Wesentlichen eine gesamte Fläche der zweiten Abschirmkomponente (10b) mit Klebstoff bedeckt ist.

9. Vorrichtung nach einem der vorhergehenden Ansprüche, wobei der zweite Teil (15) der ersten Abschirmkomponente (10a) schmaler ist als der erste Teil (13) der ersten Abschirmkomponente (10a).

10. Vorrichtung nach einem der Ansprüche 1 bis 3, wobei die zweite Abschirmkomponente eine Vielzahl separater Abdeckungskomponenten (39, 41, 43) umfasst.

11. Vorrichtung nach Anspruch 10, wobei die zweite Abschirmkomponente eine erste (39), zweite (41) und dritte (43) separate Abdeckungskomponente umfasst.

12. Vorrichtung nach Anspruch 11, wobei die erste, zweite und dritte Abdeckungskomponente (39, 41, 43) jeweils konfiguriert sind, um an den Körper eines Patienten und an den ersten Teil (13) der ersten Abschirmkomponente (10a) gekoppelt zu sein.

13. Vorrichtung nach Anspruch 12, wobei die erste, zweite und dritte Abschirmkomponente (39, 41, 43) konfiguriert sind, um sich entlang entsprechender Ränder des ersten Teils (13) der ersten Abschirmkomponente (10a) zu erstrecken, wenn sie daran gekoppelt sind.

14. Vorrichtung nach Anspruch 13, wobei die dritte Abdeckungskomponente (43) konfiguriert ist, um die erste Abdeckungskomponente (39) und die zweite Abdeckungskomponente (41) teilweise zu bedecken, wenn die erste, zweite und dritte Abdeckungskomponente an den ersten Teil (13) der ersten Abschirmkomponente (10a) gekoppelt sind.

## Revendications

1. Appareil (10) pour protéger un cathéter de tout contact avec de l'eau tombant d'une pomme de douche d'une douche, l'appareil comprenant :
un premier composant de protection (10a) comportant une première partie (13) qui est conçue pour former un premier joint d'étanchéité autour d'une ouverture de sortie (30) par laquelle un cathéter sort du corps du patient, ladite première partie (13) définissant un volume interne (17) qui s'ouvre sur le volume interne (21) d'une seconde partie de type poche (15) qui est conçue pour recevoir une partie distale dudit cathéter ; et
un second composant de protection (10b ; 39, 41, 43) destiné à adhérer au corps du patient autour d'au moins une partie de la périphérie de la première partie (13) dudit premier composant de protection (10a) de façon à former un second joint d'étanchéité contre tout contact de l'eau avec ladite ouverture de sortie (30) ;
lesdits premier (10a) et second (10b; 39, 41, 43) composants de protection coopérant, lorsque l'appareil (10) est porté par un patient, pour former deux joints d'étanchéité contre tout contact entre l'eau tombant de ladite pomme de douche et ladite ouverture de sortie (30) sans obstruer l'accès à une zone de la peau du patient en dessous de ladite seconde partie (15) dudit premier composant de protection (10a).

2. Appareil selon la revendication 1, ledit second composant de protection (10b ; 39, 41, 43) étant conçu pour recouvrir au moins partiellement la première partie (13) dudit premier composant de protection (10a).

3. Appareil selon la revendication 1 ou 2, ledit second composant de protection (10b ; 39, 41, 43) étant pourvu d'un adhésif pour former ledit second joint d'étanchéité.

4. Appareil selon la revendication 3, ledit adhésif (27) pourvu sur ledit second composant de protection (10b) étant généralement en forme de C.

5. Appareil selon la revendication 4, ledit premier joint d'étanchéité se trouvant complètement dans l'adhésif en forme de C (27) sur ledit second composant de protection (10b) lorsque le second composant (10b) est adapté sur le premier composant de protection (10a).

6. Appareil selon l'une quelconque des revendications précédentes, ladite première partie (13) dudit premier composant de protection (10a) comprenant une ouverture d'entrée (19) destinée à recevoir ledit cathéter.

7. Appareil selon la revendication 6, ledit premier composant de protection (10a) comprenant un revêtement intérieur (22) logé à l'intérieur de ladite ouverture.

8. Appareil selon l'une quelconque des revendications précédentes, pratiquement la totalité d'une face dudit second composant de protection (10b) étant recouverte d'un adhésif.

9. Appareil selon l'une quelconque des revendications précédentes, ladite seconde partie (15) dudit premier composant de protection (10a) étant plus étroite que ladite première partie (13) dudit premier composant de protection (10a).

10. Appareil selon l'une quelconque des revendications 1 à 3, ledit second composant de protection comprenant une pluralité de composants de couvercle distincts (39, 41, 43).

11. Appareil selon la revendication 10, ledit second composant de protection comprenant des premier (39), deuxième (41) et troisième (43) composants de couvercle distincts.

12. Appareil selon la revendication 11, lesdits premier, deuxième et troisième composants de couvercle (39, 41, 43) étant chacun conçus pour être couplés au corps d'un patient et à la première partie (13) dudit premier composant de protection (10a).

13. Appareil selon la revendication 12, lesdits premier, deuxième et troisième composants de protection (39, 41, 43) étant conçus pour s'étendre le long de bords respectifs de la première partie (13) du premier composant de protection (10a) lorsqu'ils sont couplés à celle-ci.

14. Appareil selon la revendication 13, ledit troisième composant de couvercle (43) étant conçu pour recouvrir partiellement le premier composant de couvercle (39) et le deuxième composant de couvercle (41) lorsque les premier, deuxième et troisième composants de couvercle sont couplés à la première partie (13) du premier composant de protection (10a).
